Europäisches Patentamt

(19) European Patent Office

Office européen des brevets

(11) Numéro de publication: **0 173 729**
**B1**

(12) **FASCICULE DE BREVET EUROPEEN**

(45) Date de publication du fascicule du brevet: **17.10.90**

(51) Int. Cl.⁵: **A 61 K 47/00**

(21) Numéro de dépôt: **85901459.9**

(22) Date de dépôt: **12.03.85**

(86) Numéro de dépôt international:
**PCT/FR85/00050**

(87) Numéro de publication internationale:
**WO 85/04106 26.09.85 Gazette 85/21**

(54) PPOCEDE DE SOLUBILISATION DE PRINCIPES ACTIFS ET COMPOSITIONS PHARMACEUTIQUES AINSI OBTENUES.

(30) Priorité: **14.03.84 CH 1279/84**

(43) Date de publication de la demande:
**12.03.86 Bulletin 86/11**

(45) Mention de la délivrance du brevet:
**17.10.90 Bulletin 90/42**

(84) Etats contractants désignés:
**AT BE CH DE FR GB LI LU NL SE**

(56) Documents cités:
**DE-A-2 102 889**
**FR-A-2 040 194**
**FR-A-2 261 011**
**GB-A-2 069 334**

**P.H List et al.: "Hagers Handbuch der Pharmazeutischen Praxis", vol. 7, part A, 4ième édition, 1971, Springer-Verlag, Berlin (DE) voir pages 216, 217, "2.OAB9. Collyria"**

**The stability of indomethacin in polyethylene glycol bases, Kahela, Kinetics of indomethacin hydrolysis, Krasowska**

(73) Titulaire: **CORBIERE, Jérôme**
**17, rue Cortambert**
**F-75016 Paris (FR)**

(72) Inventeur: **CORBIERE, Jérôme**
**17, rue Cortambert**
**F-75016 Paris (FR)**

(74) Mandataire: **Burtin, Jean-François**
**Cabinet GEFIB 55 Rue Boissonade**
**F-75014 Paris (FR)**

Courier Press, Leamington Spa, England.

EP 0 173 729 B1

# EP 0 173 729 B1

## Description

La présente invention concerne une nouvelle méthode pour rendre les produits organiques et notamment les principes actifs pharmaceutiques solubles dans l'eau ou pour accroître leur solubilité dans des milieux aqueux.

En effet les travaux antérieurs de Mack "J. Pharm. Sci. *52*, 694 (1963)", de Swarbrick" J. Pharm. Sci. *54*, 1229 (1965)" et de N. E. Webb (Bull Parenteral Drug Assoc. *30* 180 (1976)" ont souligné l'importance du problème pharmaceutique posé par l'emploi des substances hydrophobes et par la difficulté de les rendre apparemment soluble dans l'eau.

Le procédé de solubilisation que ces auteurs ont employé, utilise en général la formation de micelles ou le recours à des solvants mixtes dans lequel le principe actif est susceptible de se dissoudre.

Le problème se pose en effet d'une manière plus aigüe pour les composés hydrophobes qu'il n'est pas possible d'amener sous une forme ionisable soit un sel d'acide soit un sel de base.

En outre certains dérivés ionisables sont très facilement hydrolysables (sels de bases ou d'acides faibles) ou présentent un pH incompatible avec l'administration parentérale (solution très acides ou très alcalines).

C'est pourquoi Swarbrick a décrit une méthode générale de mise en solution dans un système aqueux qui consiste à disperser le principe actif sous forme de micelles à l'aide d'agents tensio-actifs ioniques ou non-ioniques. Ce système comporte néanmoins deux inconvénients majeurs. D'une part le principe actif est susceptible d'une altération ou d'une hydrolyse plus rapide que dans les systèmes où il est insoluble. D'autre part le principe actif perd une partie de son activité biologique soit par modification de son absorption digestive soit parce que les micelles portent une charge électrique qui modifie leur capacité de liaison au site récepteur.

C'est ainsi par exemple que les antibactériens phénoliques perdent leur activité par suite d'une interaction avec les agents tensio-actifs anioniques.

C'est pourquoi, si le système de formation de micelles permet de rendre soluble dans l'eau des composés hydrophobes, il ne résout pas le problème pharmaceutique général d'administration d'un produit soluble dont l'activité, la stabilité ou la pénétration dans l'organisme est maintenue ou même améliorée par rapport à la forme insoluble.

Plus récemment, le travail de Webb a montré la difficulté de réaliser une forme aqueuse injectable d'un composé insoluble dans l'eau, la Métiapine. La formation de cristallisations dans les formules propylène glycol/eau ou éthanol/eau, l'apparition de thrombi causée par l'injection de solution de principe actif dans de l'acide acétique rendu isotonique par du mannitol ont rendu nécessaire l'utilisation d'un autre milieu pour rendre cette molécule non ionisable, soluble dans l'eau.

Cet autre milieu consiste à dissoudre le principe actif dans un acide gras insaturé et à émulsionner cette solution lipidique dans l'eau par addition d'un agent tensio-actif non ionique. La solution qui en résulte est limpide, peut avoir un pH ajusté aux valeurs physiologiques et peut être stérilisé par filtration sur filtre stérilisant.

Cette solution provoque à l'injection l'apparition de zones hémorragiques et de plages colorées. L'analyse de ces zones à l'autopsie a mis en évidence l'existence de zones de nécrose dans les fibres musculaires et de formation de granulome dans les cellules géantes.

L'auteur a conclu que l'emploi d'un tel système solubilisant augmentait la possibilité d'irritation causée par le principe actif, en raison d'une plus grande diffusion de la solution au point d'injection par suite de la présence d'un agent tensio-actif.

Une étude semblable effectuée avec de la Terfenadine a montré la nécessité d'ajouter une quantité importante d'agent tensio-actif non ionique pour maintenir une solution claire. Elle a montré en outre les limites de concentration d'un principe actif dans de telles solutions.

C'est pourquoi ce type de dispersion huile dans l'eau n'as pas permis davantage de résoudre le problème technique de la réalisation de formes pharmaceutiques aqueuses.

Un autre exemple de réalisation de forme soluble est celui décrit par J. Gessing et P. J. Toumlin (British J. of Anaesthesia *89* (1977) 954). Le principe·actif a été mis en solution dans une émulsion lipidique (Intralipid; à 20 p.cent. Les auteurs ont constaté avec étonnement qu'avec cette nouvelle solution les quantités de principe actif nécessaires pour obtenir le même degré de sédation lors d'anesthésie médullaire, devaient être doublées (8,6 mg au lieu de 4,92 mg pour une solution aqueuse).

Les auteurs en concluent qu'il devait s'agir d'une interaction avec la pharmacocinétique du Diazépam.

D'autres références de littérature ont déjà décrit des méthodes de solubilisation particulières adaptées à un principe actif déterminé. C'est ainsi que le brevet allemand 2.102.889 décrit la préparation de solutions aqueuses contenant un mélange de sulfadimidine et de pyriméthamine. Les deux principes actifs pris isolément sont complètement insolubles dans l'eau. Cependant, la sulfadimidine se comporte comme une substance acide et est susceptible de réagir avec une base comme la pyrimethamine pour former un sel soluble.

La demande de brevet français 2.040.194 concerne également la résolution d'un problème technique distinct.

Il concerne la mise en solution de benzodiazépines, composés très peu solubles dans l'eau, en vue de la réalisation de formes injectables.

2

La benzodiazépine y est dissoute dans un polyethylène glycol à chaud (exemple I) ou bien, dans de l'éthanol puis diluée dans du polyethylène glycol et enfin, diluée avec de l'eau de manière à obtenir une préparation suffisamment fluide pour pouvoir être injectée.

En d'autres termes, ce document se rapporte à un moyen de solubilisation de benzodiazépines qui utilise du polyethylène glycol seul ou aqueux et un agent faiblement visqueux comme l'éthanol.

Il n'apparait pas que ce procédé utilise ou envisage l'emploi d'agents tampons. En outre, il semble que ce procédé ne résolve que partiellement le problème de la stabilité des solutions (voir page 5 dudit brevet).

Le brevet français 2.211.011 concerne la réalisation de solutions de chlorhydrate d'oxytetracycline -sel soluble dans l'eau- dans le polyéthylène glycol 400 auquel, on a ajouté une solution aqueuse d'un sel de Magnesium et une solution d'éthanolamine. En effet, les solutés de tétracycline et de ses dérivés sont peu stables à l'état simple mais sont beaucoup plus stables sous forme de chélates magnesiens. Cependant, la formation du complexe magnesien amène la libération d'acide chlorhydrique qui acidifie le milieu; l'alcalinisation par l'éthanolamine (base forte) a pour objet de neutraliser l'acide chlorhydrique libéré d'une part et d'amener la solution à un pH basique où le complexe magnesien est plus stable d'autre part.

Enfin, en ce qui concerne l'utilisation de milieux ou de substances tampon, il convient de considérer que ces systèmes sont décrits dans la littérature (cf. Hagers Handbuch der pharmazeutischen Praxis 4ème ed. (1971) 216—217), mais jouent un rôle complètement différent. Dans la plupart des collyres, on prépare un soluté dont on ajuste le pH à des valeurs physiologiques, compatibles avec l'administration oculaire, avec un système tampon dont le pH est lui-même équivalent à celui du milieu oculaire.

Dans le présent procédé, le système tampon joue un double rôle. Il contribue d'une part à mettre en solution le principe actif dispersé au préalable dans un polyéthylène glycol et d'autre part, à amener le milieu à un pH où la molécule chimique est stable. En effet, il est aussi important de conserver l'activité thérapeutique du principe actif que de maintenir le pH du milieu à une valeur compatible avec la tolérance locale.

Finalement, on peut estimer que le problème de la solubilisation de principes actifs hydrophobes dans un milieu aqueux présente deux facettes:

une facette physico-chimique:
la solution doit être stable, à un pH compatible avec l'injection ou l'administration et le principe actif ne doit pas être dégradé par la mise en solution.

une facette biologique:
la solution doit conserver toute l'activité pharmacologique ou clinique du principe actif et ne pas modifier les caractéristiques d'absorption ou de pharmacocinétiques dans un sens défavorable.

C'est pourquoi la méthode de solubilisation, objet de la présente demande de brevet, apporte une solution nouvelle et inattendue au problème déjà posé depuis de nombreuses années et non encore résolu.

L'invention concerne spécifiquement un procédé de préparation de solutés aqueux de molécules médicamenteuses hydrophobes, aisément hydrolysables ou oxydables, destinés à l'administration par voie parentérale, digestive ou permuqueuse caractérisé en ce que la substance médicamenteuse est dispersée dans un ou plusieurs polymères d'éthylène glycol anhydre dont le poids moléculaire est compris entre 300 et 700, puis diluée consécutivement ou extemporanément avec un système tampon dont le pH est fixé entre 4,5 et 8 de manière à réaliser un soluté aqueux stable à la conservation.

Il s'agit d'une méthode par co-solvant dans lequel la substance médicamenteuse est dispersée dans un ou plusieurs polymères d'éthylène glycol de poids moléculaire peu élevé en dehors de toute addition d'eau puis la pseudo-solution résultante est mêlée à un milieu aqueux de pH déterminé.

L'invention s'applique particulièrement au problème de la préparation de solutés aqueux à base de N-acyl Indoles comme l'Indométacine, la Cinmétacine, l'Acémétacine, la nicamétacine ou la sermétacine. Les composés présentent une fonction N-acyle particulièrement labile et l'hydrolyse de ce groupement en milieu neutre ou faiblement basique entraine la perte de l'activité, comme c'est le cas dans la plupart des opérations de salification avec une base forte.

Ce milieu aqueux de pH déterminé est de préférence une solution d'un tampon dont la valeur du pH est déterminée en fonction des caractéristiques physico-chimiques de la molécule de principe actif. Selon un mode d'exécution préféré le système tampon selon l'invention assure un pH compris entre 5,5 et 7,0.

On peut estimer que la dispersion du principe actif dans le polyéthylène glycol de haut poids moléculaire amène la formation de particules micellaires où chaque molécule de principe acif est protégée chimiquement par les molécules de polyéthylène glycol et qu'ainsi, les fonctions chimiques réactives du principe actif sont bloquées.

Il en résulte que le principe actif est à l'abri des effets de l'hydrolyse ou de l'auto-oxydation.

L'invention s'applique également à la réalisation de milieux aqueux dans lesquels la concentration de principe actif est élevée ou de milieux aqueux présentant un petit volume.

Dans un mode d'exécution préféré, l'invention peut être définie en ce que le principe actif est dissout au préalable dans un ou plusieurs polyéthylène glycols de haut poids moléculaire compris entre 300 et 700 comme par exemple le polyéthylène glycol 400 ou le polyéthylène glycol 600 puis d'ajouter la phase aqueuse dont le pH est ajusté.

La phase organique peut être additionnée d'agents de conservation, de stabilisation, d'agents

3

aromatiques, de colorants, d'agents de sapidité.

La phase aqueuse renferme outre l'agent déterminant le pH, des sels minéraux, des agents de conservation ou des principes actifs complémentaires solubles dans l'eau.

Parmi les agents de conservation solubles ou miscibles à la phase organique, on pourra citer les p. hydroxybenzoate de méthyle ou de propyle, le gallate d'isopropyle ou le terbutyl p-hydroxy anisole.

Parmi les sels minéraux que l'on ajoute à la phase aqueuse, on pourra citer ceux nécessaires pour assurer l'isotonie de la solution aqueuse comme par exemple le chlorure de sodium ou le chlorure de lithium.

L'agent déterminant le pH est de préférence un système tampon comme par exemple le mélange phosphate monosodique phosphate disodique, le mélange acide borique—borate de sodium ou le mélange acide acétique—acétate de sodium. Le choix d'un tel système dépend essentiellement de la nature du principe actif et de sa voie d'administration. L'administration par voie digestive permet d'administrer des solutés dont le pH est compris entre 4,5 et 8.

L'administration par voie parentérale permet l'utilisation de solutés dont le pH est compris entre 5,5 et 7,5.

L'administration par voie muqueuse ou transdermique nécessite des pH compris entre 5,5 et 7,0.

Le choix du système tampon est conditionné par la compatibilité et l'efficacité de la voie d'administration.

D'une manière préférée, le système tampon est un de ceux adaptés à l'administration par voie muqueuse.

La nature du produit organique hydrophobe incorporé à de telles préparations est extrêmement extensive. Il peut s'agir d'un stéroïde comme les composés hormonaux (Estradiol, Estrone, Testostérone, Propionate de Testostérone, Acétate de Noréthynodiol, Acétate de Trenbolone, Ethynyl Estradiol). Il peut s'agir d'agents antalgiques comme le Paracétamol, le Bénorylate, la Glafénine, la Floctafénine, la Nicafénine ou l'Anthrafénine. Il peut s'agir d'agents anti-inflammatoires comme le Piroxicam, l'oxicam, le Ténoxicam, l'Ibuprofène, le Flurbiprofène, l'acide tiaprofènique ou le Diflunisal.

Il peut s'agir d'agents uricosuriques comme l'Allopurinol, la Benzbromarone ou la Bromarone.

Il peut s'agit d'agents cardio-vasculaires comme la Khelline, l'Amiodarone, l'Amrinone ou l'Erythrite pentanitrate.

Il peut s'agir d'agents diurétiques comme le Chlorothiazide, le Cyclothiazide, l'Acide Ethacrynique ou le Triamtérène.

Il peut s'agir d'agents antibactériens comme les Sulfamides, le Triméthoprim, les Pénicillines semi-synthétiques, les Macrolides estérifiés ou salifiés comme le Dipropionate d'Erythromycine ou de Josamycine, la Phosphonomycine, les Tétracyclines, les Enniatines, les Rifampicines ou les Céphalosporines.

Il peut s'agir d'agents hypnotiques ou anti-épileptiques comme les Barbituriques, les Urées ou les Oxazolidones.

Il peut s'agir de médicaments anti-dépresseurs comme l'Imipramine, la Clomipramine, la desméthylchlorimipramine, la Miansérine ou l'Amineptine.

Il peut s'agir de composés neuroleptiques comme l'Haloperidol, la Dompéridone, la Chlorprométhazine, le Métoclopramide, le Sulpiride, le Diazépam, le Lorazépam, le Bromazépam ou le Nitrazépam.

Il peut s'agir d'agents anti-fongiques ou anti-Trichomonas comme le Ternidazole, le Secnidazole, le Miconazole, l'Econazole ou le Clotrimazole.

Il peut encore s'agir d'agents anti-viraux comme l'Iodo désoxy uridine ou la Citarabine ou d'agents anti-mitotiques comme le Ftorafur ou le Carmofur.

La concentration en principe actif dans de telles préparations s'échelonne entre 0,1 et 25% et de préférence entre 1 et 10%.

L'invention concerne encore spécifiquement un procédé de réalisation de formes pharmaceutiques aqueuses destinées à l'usage externe comme par exemple les gouttes nasales ou auriculaires ou les collyres ou les solutés caractérisé en ce que le principe actif est dissous dans un polyéthylène glycol de poids moléculaire compris entre 300 et 700, additionné ou non de polyéthylène glycol de haut poids moléculaire, puis dispersé dans une solution aqueuse dont le pH est fixé. La solution résultante peut être répartie en doses unitaires ou lyophylisées de manière à pouvoir être remise en solution au moment de l'emploi.

Le principe actif peut également être seulement dissous dans le polyéthylène glycol de poids moléculaire compris entre 300 et 700, et additionné de la phase aqueuse extemporanément, au moment de l'emploi. La phase aqueuse peut alors renfermer en solution un autre principe actif parfaitement soluble dans l'eau. C'est le cas pour les gouttes nasales ou pour les collyres. Il est préférable de dissoudre dans le polyéthylène glycol un principe actif insoluble dans l'eau comme par exemple un agent anti-inflammatoire et de dissoudre dans la phase aqueuse un autre principe actif comme un agent décongestionnant, par exemple la Néosynéphrine ou le Chlorhydrate de Phenylpropanolamine ou comme un agent anti-bactérien par exemple la Chlorhexidine. Le mélange des deux solutés se fera extemporanément et permettra ainsi l'administration simultanée de deux principes actifs d'action complémentaire ou synergique.

Les exemples suivants illustrent l'invention sans toutefois la limiter.

Exemple I

Collyre a l'indometacine

Soluté A

| Indométacine | 0,4 g |
| p. Hydrobenzoate de méthyle | 0,2 g |
| Polyéthylène glycol 400 | 99,4 g |

Soluté B

| Acide Borique . | 1,1774 g |
| Chlorure de sodium | 0,2769 g |
| Borate de sodium | 0,09088 g |
| Eau q.s.p. | 100 ml |

Les deux solutés sont mélangés. La solution résultante est parfaitement stable 4 mois à température ordinaire.

Exemple II

Solute concentre d'indometacine

Soluté A

| Indométacine | 0,0005 g |
| p-Hydroxybenzoate de méthyle | 0,0025 g |
| Polyéthylène glycol 400 | 3,85 ml |

Le soluté A est réparti en flacons de 5 ml à raison de 1,15 ml par flacon.

Soluté B

| Acide borique | 0,04533 g |
| Chlorure de sodium | 0,01066 g |
| Borate de sodium | 0,003498 g |
| Eau q.s.p. | 3,85 ml |

Le soluté B est réparti en flacons de 5 ml à raison de 3,85 ml de soluté B par flacon. Le soluté A placé à 25°C pendant 5 mois conserve 99,8 p. cent de sa teneur en principe actif.

Le tableau, ci-joint, fournit les données sur la conservation des deux solutés d'Indométacine.

TABLEAU I

Teneur en Indométacine 0,1 et 0,5%
T=0    0,1%    0,0997 g % en Indométacine
T=0    0,5%    0,498  g % en Indométacine

R=Reconstitué
NR=Non reconstitué
Pas de conservateur.

| | R (0,1 g %) | | N R (0,5 g %) | |
| --- | --- | --- | --- | --- |
| | 25°C | 40°C | 4°C | 25°C |
| 1 mois | 0,0988 | 0,0941 | | |
| 2 mois | 0,0946 | 0,0866 | | |
| 3 mois | 0,0930 | | | |
| 4 mois | | | 0,0968 | |
| 5 mois | | | | 0,488 |

Exemple III

Solute de theophylline
Soluté A

| Théophylline | 4,50 g |
| P. Hydroxybenzoate de propyle | 0,001 g |
| Polyéthylène glycol 600 | 95,5 ml |

Soluté B

| Phosphate monopotassique | 0,68 g |
| Phosphate disodique | 0,71 g |
| Eau q.s.p. | 100 ml |

Les deux solutions sont mélangées pour obtenir un soluté de Théophylline à 0,225 g pour 10 ml.

Exemple IV

Solute d'hesperidine
Soluté A

| Hespéridine | 10 g |
| Gallate d'isopropyle | 0,002 g |
| Polyéthylène glycol 300 | 90 ml |

Soluté B

| Citrate monosodique | 0,20 g |
| Citrate disodique | 0,222 g |
| Arôme citron | 0,01 ml |
| Eau q.s.p. | 100 ml |

Les solutions A et B sont mélangées pour obtenir un soluté buvable contenant 0,05 g d'Hesperidine par ml.

## Revendications

1. Procédé de préparation de solutés aqueux de molécules médicamenteuses hydrophobes aisément hydrolysables ou oxydables destinés à l'administration par voie parentérale, digestive ou permuqueuse caractérisé en ce que la substance médicamenteuse est dispersée dans un ou plusieurs polymères d'éthylène glycol anhydre dont le poids moléculaire est compris entre 300 et 700, puis, dilué consécutivement ou extemporanément avec un système tampon dont le pH est fixé entre 4,5 et 8 de manière à réaliser un soluté aqueux stable à la conservation.

2. Procédé de préparation de solutés aqueux selon la revendication 1 dans lequel, la substance médicamenteuse hydrophobe est un N-acyl Indole choisi dans le groupe constitué par l'indoméactine, la cinmétacine, l'acémétacine, la nicamétacine et la sermétacine.

3. Procédé de préparation de solutés aqueux selon la revendication 1 ou la revendication 2 dans lequel le système tampon assure un pH compris entre 5,5 et 7,0.

4. Procédé selon l'une des revendications 1 à 3 pour la préparation de solutés aqueux de molécules médicamenteuses hydrophobes dans lequel le système tampon est adapté à l'administration par voie muqueuse.

5. Les compositions pharmaceutiques renfermant un soluté aqueux de molécules médicamenteuses hydrophobes obtenu selon l'une des revendications 1 à 4.

6. Les compositions pharmaceutiques selon la revendication 5 présentées sous forme de collyre ou de solutés pour l'usage oculaire.

7. Les compositions pharmaceutiques selon l'une des revendications 5 et 6 dans lesquelles, la teneur du principe actif hydrophobe est comprise entre 0,1 et 25%.

8. Les compositions pharmaceutiques selon l'une des revendications 5 et 6 dans lesquelles, le principe actif hydrophobe est un N-acyl Indole comme défini à la revendication 2.

9. Les compositions pharmaceutiques selon l'une des revendications 5 à 8 dans lesquelles, la teneur en indométacine par prise unitaire est comprise entre 0,1 et 1%.

## Patentansprüche

1. Verfahren zur Bereitung von wässrige Lösungen von hydrophoben arzneilische Molekülen die leicht hydrolysierbar oder Sauerstoffempfindliche sind, die sich für die durch parenterale, Verdauung oder permukosale Weg Verabreichung, Vorbereiten sind, dadurch gekennzeichnet ist, dass die arzneilige Verbindung in einem oder mehreren wasserfreien Polymeren von Ethylenglycol dessen Molekular Gewicht zwischen 300 und 700 liegt, veiteilt wird, denn anschließend oder gleichzeitig mit einem Puffersystem, dessen pH Wert zwischen 4,5 und 8 aufgestellt ist, verdünnt wird um eine wässerige Lagerungsstabile Lösung zu gewinnen.

2. Verfahren zur Bereitung von wässerige Lösungen nach Anspruch 1, worin die hydrophobe arzneilische Verbindung ein N-acyl Indol ist, das aus die Gruppe von Indometacin, Cinmetacin, Acemetacin, Nicametacin und Sermetacin gewählt ist.

3. Verfahren zur Bereitung von wässerige Losungen nach Anspruch 1 oder Anspruch 2, worin der Puffersystem ein pH Wert von 5,5 bis 7,5 bewährt.

4. Verfahren nach Ansprüche 1 bis 3 zur Bereitung von Lösungen von hydrophoben arzneiligen Verbindungen, worin der Puffersystem sich für die Abreichung durch die Schleimhaut weg eignet.

5. Die pharmazeutische Zubereitungen die eine nach eine der Ansprüche 1 bis 4 gewonnene wässerige Lösungen von hydrophoben arzneilige Verbindungen, enthalten.

6. Die pharmazeutische Zubereitungen nach Anspruch 5, in der Form von Augentropfen oder Lösungen für Augenanwendung.

7. Die pharmazeutische Zubereitungen nach einem der Ansprüche 5 und 6, worin das Gehalt von hydrophoben Wirkstoff zwischen 0,1 und 25% liegt.

8. Die pharmazeutische Zubereitungen nach einer der Ansprüche 5 und 6 worin der hydrophobe Wirkstoff aus einem als in Anspruch 2 bestimmte N-acyl Indol besteht.

9. Die pharmazeutische Zubereitungen nach einem der Ansprüche 5 und 6 worin das Gehalt an Indomethacin zwischen 0,1 und 1% bei einzelnem Dosis liegt.

**Claims**

1. A process for preparing aqueous solutes of hydrophobic pharmaceutical molecules which are easily hydrolysed or oxydized, intended for administration through parenteral, digestive or permucous ways of administration, characterized in that the pharmaceutical compound is dispersed into one or several anhydrous polymers of ethylene glycol, the atomic weight of which ranges from 300 to 700 then is diluted consecutively or extemporaneously, with a buffer system the pH of which is adjusted between 4.5 and 8 in order to realize an aqueous solute, stable to the storage.

2. A process for preparing aqueous solutes according to claim 1 in which the hydrophobic pharmaceutical compound is a N-acyl Indole selected from the group consisting of Indomethacin, Cinnetacin, Acemetacin, Nicametacin, and Sermetacin.

3. A process for preparing aqueous solutes according to claim 1 or claim 2 wherein the buffer system provides a pH value ranging from 5,5 to 7.

4. A process according to any of claims 1 to 3 for preparing aqueous solutes of hydrophobic pharmaceutical compounds, wherein the buffer system is adapted for the administration through the mucosal way.

5. The pharmaceutical compositions containing an aqueous solute of hydrophobic pharmaceutical compounds whenever obtained in accordance with any of claims 1 to 4.

6. The pharmaceutical compositions according to claim 5 dispensed in the form of eye drops or solutions for ophthalmic use.

7. The pharmaceutical compositions according to any of claims 5 and 6 wherein the content of hydrophobic active ingredient ranges from 0.1 to 25%.

8. The pharmaceutical compositions according to any of claims 5 and 6 wherein the hydrophobic active ingredient is a N-acyl Indole as defined in claim 2.

9. The pharmaceutical compositions according of any of claims 5 to 8 wherein the content in Indomethacin per unit dosage ranges from 0.1 to 1%.